# EUROPÄISCHE PATENTANMELDUNG

(11) **EP 4 310 851 A1**
(43) Veröffentlichungstag der Anmeldung: **24.01.2024**
(21) Anmeldenummer: 23185024.9
(22) Anmeldetag: 12.07.2023
(51) Int. Cl.: G16H 20/17, G16H 40/63

(54) **VERFAHREN ZUR REDUZIERUNG EINES BOLUS, PROGNOSEVERFAHREN, SICHERHEITSVORRICHTUNG UND MEDIZINISCHE PUMPE**

(30) Priorität: 20.07.2022 DE 102022118179
(71) Anmelder: B. Braun Melsungen AG, 34212 Melsungen (DE)
(72) Erfinder: OBERMANN, Dennis, 34628 Willingshausen (DE); WIEGAND, Thomas, 34576 Homberg (Efze) (DE); MORITZEN, Hans-Christian, 34119 Kassel (DE)
(74) Vertreter: Winter, Brandl - Partnerschaft mbB

(57) **Zusammenfassung**

Die vorliegende Offenbarung betrifft ein Verfahren zur Reduzierung eines Bolus in einer Fluidleiteinheit, insbesondere einer Ausgangsleitung, einer medizinischen Pumpe (1), vorzugsweise einer Infusionspumpe, mit den folgenden Schritten. Erfassen eines Druckverlaufs in der Fluidleiteinheit durch eine Sensoreinheit (4); Speichern des erfassten Druckverlaufs in einer Speichereinheit (6); Ermitteln einer Okklusionswahrscheinlichkeit durch eine Steuereinheit (8) basierend auf dem erfassten Druckverlauf; Erfassen eines von der Pumpe (1) geförderten Volumens für einen Betriebsbereich mit hoher Okklusionswahrscheinlichkeit, insbesondere einer Anzahl an Motorschritten eines Pumpenmotors oder eines Drehwinkels des Pumpenmotors, durch die Steuereinheit (8); Detektieren eines tatsächlichen Auftretens einer Okklusion zu einem Detektionszeitpunkt durch eine Detektionseinheit (10) bei Überschreiten eines Druckgrenzwertes; und Zurückpumpen des für den ermittelten Betriebsbereich mit hoher Okklusionswahrscheinlichkeit erfassten von der Pumpe (1) geförderten Volumens durch die Pumpe (1), insbesondere durch Zurückfahren der erfassten Motorschritte oder des erfassten Drehwinkels. Ferner betrifft die Offenbarung ein Prognoseverfahren, eine Sicherheitsvorrichtung (2) und eine medizinische Pumpe (1) gemäß den nebengeordneten Ansprüchen.

## Beschreibung

### Technisches Gebiet

Die vorliegende Offenbarung betrifft ein Verfahren zur Reduzierung eines Bolus in einer Fluidleiteinheit, insbesondere in einer Ausgangsleitung, einer medizinischen Pumpe, vorzugsweise einer Infusionspumpe. Daneben betrifft die vorliegende Offenbarung ein Prognoseverfahren zur Ermittlung einer Okklusionswahrscheinlichkeit, eine Sicherheitsvorrichtung zur Reduzierung eines Bolus und eine medizinische Pumpe, insbesondere eine Infusionspumpe gemäß den Oberbegriffen der nebengeordneten Ansprüche.

### Hintergrund der vorliegenden Offenbarung

Bei medizinischen Pumpen, insbesondere Infusionspumpen, können Okklusionen bzw. Verstopfungen / Verschlüsse auftreten. Okklusionen können beispielsweise durch ein Knicken oder Verdrehen von elastischen Schläuchen auftreten, etwa ungewollt durch eine entsprechende Handhabung. Dabei ist insbesondere eine Okklusion in einer Fluidleiteinheit, insbesondere einer Ausgangsleitung, der Pumpe, also einem Abschnitt in welchem das Fluid an den Patienten gefördert wird, kritisch. Durch die Okklusion kann sich insbesondere Infusionsflüssigkeit in der elastischen Ausgangsleitung sammeln, die als Wegwerfartikel ausgeführt ist. Die aufgrund der Okklusion angesammelte medizinische Flüssigkeit stellt dabei einen Bolus dar. Wenn die Okklusion beseitigt, also das Hindernis für das Fluid entfernt ist, kann das angesammelte Volumen an Infusionsflüssigkeit ungünstiger Weise dem Patienten auf einmal verabreicht werden. Diese intensive und schnelle Gabe kann insbesondere bei kritischen Medikamenten für den Patienten schädlich und im Extremfall sogar letal sein. Ferner kann durch die Okklusion der Druck in der Fluidleiteinheit über ein gewünschtes Sicherheitslevel hinaus ansteigen, was ebenso zu Schäden führen kann.

Es ist daher bekannt, medizinische Pumpen mit einer Okklusionserkennung bzw. einer Einheit zur Okklusionserkennung auszustatten. Die Okklusionserkennung basiert dabei auf Druckmesswerten von Drucksensoren. Im Falle einer Okklusion in der Ausgangsleitung der Pumpe steigt der Druck in der Ausgangsleitung entsprechend an. Der oder die Drucksensoren erkennen den Druckanstieg und schalten die Pumpe schnellstmöglich ab. Um zu verhindern, dass die angesammelte Infusionsflüssigkeit dem Patienten ungewollt verabreicht wird, weisen bekannte Pumpen mit Okklusionserkennung eine Funktion zur sogenannten Bolusreduzierung /Reduzierung eines Bolus auf. Dabei wird ein festes/fixes Volumen, das basierend auf einem Druckgrenzwert, neuerdings gar zusammen mit einem Kennwert/Parameter des Wegwerfartikels, berechnet wird, abgepumpt. Dahinter steckt die Annahme, dass der Aufbau der Ausgangsleitung, d.h. die Zusammensetzung der verschiedenen Wegwerfartikel immer gleich für verschiedene Anwendungen ist und sich auch die Okklusion immer am gleichen Ort der Ausgangsleitung befindet. Es wird gewissermaßen ein einziger fester Wert für den Wegwerfartikel und die Pumpe festgesetzt und in allen Situationen einer Okklusion dieser feste, standardisierte Wert verwendet.

Die Erfahrung in der Praxis hat jedoch gezeigt, dass solche Annahmen nicht zutreffen. Das Bolusvolumen hängt nämlich nicht nur von einem Druck in der Leitung ab, sondern von weiteren Faktoren wie dem Material der Wegwerfartikel, der Geometrie der Wegwerfartikel, dem Aufbau der klinischen Anordnung, der Umgebungstemperatur und insbesondere auch der Stelle bzw. dem Ort der Okklusion. Es ist also nicht ausreichend, lediglich ein festes Volumen an gefördertem Fluid zurück zu pumpen.

Aus dem Stand der Technik sind Lösungen bekannt, die den Zeitpunkt eines Auftretens der Okklusion schätzen und basierend auf diesem geschätzten Zeitpunkt eine Schätzung dahingehend abgeben, welches Volumen der Bolus aufweisen könnte. Das geschätzte Volumen wird daraufhin von der Pumpe zurückgepumpt.

### Stand der Technik

Aus der EP 1 136 089 B1 ist ein Verfahren zur Analyse von Druckänderungen in einer Infusionsvorrichtung bekannt. Die Infusionsvorrichtung weist mehrere Module auf. Wenn eine Druckänderung in einer Leitung erkannt wird, wird eine Analyse durchgeführt, um eine Verwicklung anderer Module in die Druckänderung zu bestimmen. Ein Druckverlauf wird retrospektiv analysiert, um den Zeitpunkt des Auftretens einer Okklusion zu bestimmen. Dabei weisen insbesondere Druckänderung auf das Auftreten der Okklusion hin. Eine Zeitspanne zwischen einem Auftrittszeitpunkt der Okklusion und einem Detektionszeitpunkt der Okklusion wird ermittelt. Nach der Detektion der Okklusion wird Infusionsfluid diese Zeitspanne lang zurückgepumpt. Dadurch wird der Bolus reduziert.

Die WO 2021 / 042 126 A1 offenbart ein System zur Reduzierung eines Bolus nach einer Detektion einer Okklusion. Dabei wird Fluid nach der Detektion der Okklusion zurückgepumpt, bis ein vorbestimmtes, sicheres Druckniveau erreicht wird, von dem angenommen wird, dass die Okklusion beseitigt ist. Diese Reduzierung des Bolus ist jedoch sehr ungenau.

Allen bekannten Lösungen haben jedoch den gemeinsamen Nachteil, dass aktuell die Schätzung des Bolusvolumens sehr ungenau ist. Daher kann die Pumpe im ungünstigen Fall das falsche Volumen zurückpumpen, so dass entweder zu viel Fluid abgepumpt wird oder ein Bolus in der Fluidleiteinheit, etwa der Ausgangsleitung, verbleibt.

### Zusammenfassung der Offenbarung

Es sind also die Aufgaben der vorliegenden Offenbarung, die Nachteile des Stands der Technik zu überkommen oder zumindest zu verringern und insbesondere ein Verfahren, ein Prognoseverfahren, eine Sicherheitsvorrichtung und eine medizinische Pumpe bereitzustellen, mit denen bei Auftreten einer Okklusion ein Volumen eines (ungewollten) Bolus sicher und zuverlässig reduziert oder zurückgesetzt werden kann. Eine Teilaufgabe kann darin gesehen werden, insbesondere ein Volumen eines Fluides zu erfassen, das nach Auftreten einer Okklusion gefördert wurde, und dieses Volumen entsprechend zurückzupumpen.

Diese Aufgaben der vorliegenden Offenbarung werden hinsichtlich eines Verfahrens erfindungsgemäß durch die Merkmale gemäß Anspruch 1 gelöst. Die Aufgaben der vorliegenden Offenbarung werden ferner hinsichtlich eines Prognoseverfahrens erfindungsgemäß durch die Merkmale gemäß Anspruch 6 gelöst, hinsichtlich einer Sicherheitsvorrichtung erfindungsgemäß durch die Merkmale gemäß Anspruch 7 und hinsichtlich einer medizinischen Pumpe erfindungsgemäß durch die Merkmale gemäß Anspruch 12 gelöst.

Die vorliegende Offenbarung betrifft ein Verfahren zur Reduzierung eines (ungewollten) Bolus bzw. einer Ansammlung eines Fluides in einer Fluidleiteinheit (stromabwärts der Pumpe), insbesondere einer Ausgangsleitung, einer medizinischen Pumpe, vorzugsweise einer Infusionspumpe. Das offenbarungsgemäße Verfahren weist die folgenden Schritte auf. Eine Sensoreinheit erfasst, insbesondere kontinuierlich oder wiederholend in diskreten Zeitabständen, einen Druckverlauf in der Fluidleiteinheit, insbesondere in der Ausgangsleitung. Eine Speichereinheit speichert vorzugsweise den erfassten Druckverlauf, um diesen auch zu einem späteren Zeitpunkt insgesamt analysieren zu können und eine zeitliche Änderung zu bestimmen. Eine Steuereinheit ermittelt eine Okklusionswahrscheinlichkeit, also eine Wahrscheinlichkeit zwischen 0% und 100%, dass eine Okklusion auftritt, basierend auf dem erfassten Druckverlauf (Der zeitlich erfasste Druckverlauf kann also dafür herangezogen werden, um durch eine entsprechende Analyse eine Wahrscheinlichkeit der Okklusion zu bestimmen. Für einen Betriebsbereich der Pumpe mit hoher Okklusionswahrscheinlichkeit (also einer Okklusionswahrscheinlichkeit über einem vordefinierten Grenzwert, der einen Betriebsbereich einer niedrigen Okklusionswahrscheinlichkeit von einer hohen Okklusionswahrscheinlichkeit trennt) erfasst die Steuereinheit (ab diesem Zeitpunkt des Wechsels in den Betriebsbereich der hohen Okklusionswahrscheinlichkeit) ein von der Pumpe gefördertes Volumen. Insbesondere wird eine Anzahl an getätigten Motorschritten eines Pumpenmotors oder ein Drehwinkel des Pumpenmotors oder eine Anzahl an Umdrehungen der Pumpe bzw. Pumpenmotors erfasst. Sollte die medizinische Pumpe Fluid mithilfe einer Peristaltik fördern, kann auch eine Peristaltikbewegung erfasst werden. Eine Detektionseinheit detektiert/bestimmt ein Auftreten einer Okklusion zu einem Detektionszeitpunkt (also nach einem Auftrittszeitpunkt) bei Überschreiten einer Alarmbedingung, insbesondere eines Druckgrenzwertes als Alarmbedingung. Das für den ermittelten Betriebsbereich mit hoher Okklusionswahrscheinlichkeit erfasste von der Pumpe geförderte Volumen wird von der Pumpe dann entsprechend wieder zurück gepumpt. Insbesondere werden dabei die erfassten Motorschritte oder der erfasste Drehwinkel von dem Pumpenmotor zurückgefahren.

Der Betriebsbereich mit der hohen Okklusionswahrscheinlichkeit wird basierend auf dem erfassten Druckverlauf ermittelt. Wenn die Pumpe in dem Bereich hoher Okklusionswahrscheinlichkeit betrieben wird (bzw. in diesen Bereich wechselt), werden (ab diesem Zeitpunkt des Betriebsbereichs hoher Okklusionswahrscheinlichkeit) die Motorschritte des Pumpenmotors oder die Anzahl der Umdrehungen oder ähnliches erfasst. Die Anzahl Umdrehungen oder Motorschritte oder Drehwinkel werden vorzugsweise von der Steuereinheit erfasst und in der Speichereinheit gespeichert (etwa eine Anzahl an Umdrehungen über die Zeit aufgezeichnet). Wenn die Detektionseinheit zu einem Detektionszeitpunkt eine Okklusion detektiert, kann vorzugsweise ein Alarm oder Ähnliches ausgegeben werden und die Pumpe wird gestoppt. Die erfassten Motorschritte oder Umdrehungen entsprechen mit einer bestimmten Wahrscheinlichkeit den Schritten, die getätigt wurden, seitdem die Okklusion besteht (also seit dem Auftrittszeitpunkt der Okklusion), aber noch nicht detektiert worden ist (also noch vor dem Detektionszeitpunkt). Somit können die Motorschritte oder der Drehwinkel zwischen dem Auftreten und der Detektion der Okklusion ermittelt oder zumindest geschätzt werden, ohne den exakten Zeitpunkt des Auftretens der Okklusion zu kennen. Eine Zeitspanne zwischen dem Auftreten und der Detektion der Okklusion ist eine Zeitspanne zwischen dem Auftrittszeitpunkt der Okklusion und dem Detektionszeitpunkt der Okklusion. Die erfassten Motorschritte bzw. Anzahl Umdrehungen bzw. Drehwinkel werden nach der Detektion der Okklusion zum Detektionszeitpunkt vom Pumpenmotor wieder zurückgefahren, so dass der ungewollte Bolus wieder aus der Fluidleiteinheit, insbesondere der Ausgangsleitung der Pumpe, sicher entfernt wird.

Die Okklusionswahrscheinlichkeit ist dabei eine Wahrscheinlichkeit (zwischen 0% und 100%), mit der eine Okklusion auftritt und wird von der Steuereinheit basierend auf dem erfassten Druckverlauf ermittelt. Dabei kann eine hohe Okklusionswahrscheinlichkeit vorliegen, wenn die Wahrscheinlichkeit für das Auftreten einer Okklusion, die von der Steuereinheit ausgegeben wird, höher als ein Grenzwert, beispielsweise 50%, vorzugsweise höher als 70%, ist. Insbesondere kann der vordefinierte Grenzwert vor Beginn einer Behandlung variabel einstellbar sein. Es ist aber auch andere Grenzwert für einen Wechsel des Betriebsmodus von der niedrigen zur hohen Okklusionswahrscheinlichkeit denkbar, solange diese einen positiven Wert haben, also auch etwa 20%. Wenn nun vorliegend eine Okklusionswahrscheinlichkeit geschätzt wird und diese einen Grenzwert oder eine Alarmbedingung überschreitet, so kann hierüber in gewisser Weise direkt ein Auftreten einer Okklusion bestimmt werden. Es muss also, im Gegensatz zum Stand der Technik, nicht mehr von einem Detektionszeitpunkt relativen von diesem aus retrospektiv zurückgegangen werden, sondern es werden sozusagen zwei Zeitpunkte bestimmt, einen (geschätzten) Auftrittszeitpunkt (also ein Betriebsbereich hoher Okklusionswahrscheinlichkeit) und einen Detektionszeitpunkt und bereits ein Pumpvolumen für diesen Zeitbereich individuell ermittelt. So kann noch genauer ein Bolusvolumen ermittelt und zurückgepumpt werden.

Die Steuereinheit gibt beispielsweise ein Steuersignal aus, das den Pumpenmotor veranlasst, die erfassten Motorschritte oder den erfassten Drehwinkel oder die erfasste Anzahl an Umdrehungen zurückzufahren.

Die Alarmbedingung oder der Grenzwert bei dessen Überschreiten eine Okklusion detektiert wird, kann beispielsweise ein (vorbestimmter) Druckgrenzwert sein. Die Drucksensoren sind bereits in der Fluidleitung vorhanden. Ferner kann vorzugsweise auch das Überschreiten einer vordefinierten Okklusionswahrscheinlichkeit das Auftreten der Okklusion anzeigen. Dabei muss insbesondere die Okklusionswahrscheinlichkeit für die Detektion noch höher als die hohe Okklusionswahrscheinlichkeit sein. Das Auftreten einer Okklusion kann vorzugsweise auch durch das Überschreiten einer bestimmten Anzahl an Motorschritten und/oder einer bestimmten Zeit detektiert werden, vorzugsweise, wenn die Pumpe eine bestimmte Zeit gefördert hat, ohne dass ein Volumenstrom resultiert.

Zusammenfassend liegt der Kern der Offenbarung darin, den (Betriebs-)Modus mit hoher Okklusionswahrscheinlichkeit zu erkennen (und ein Auftreten einer Okklusion gewissermaßen vorherzusagen), in bzw. ab diesem Modus (der hohen Okklusionswahrscheinlichkeit) das Pumpvolumen, insbesondere die Motorschritte des Pumpenmotors, zu erfassen und bei der (tatsächlichen) Detektion der Okklusion die erfassten Motorschritte zwischen dem Auftritts- und Detektionszeitpunkt der Okklusion zurückzufahren und somit das von der Pumpe zwischen dem Auftreten und der Detektion der Okklusion geförderte Volumen zurück zu pumpen.

Durch das offenbarungsgemäße Verfahren kann das Bolusvolumen sehr genau geschätzt werden. Dadurch kann die Pumpe das Bolusvolumen sehr zielgenau abpumpen. Durch das Signal, das die Okklusionswahrscheinlichkeit von gering/niedrig zu hoch wechselt, wird die Erfassung des Pumpvolumens, insbesondere der Motorschritte, gestartet. Der Wechsel des Betriebsmodus von der niedrigen zu der hohen Okklusionswahrscheinlichkeit entspricht mit einer Wahrscheinlichkeit (der Okklusionswahrscheinlichkeit), dem Auftrittszeitpunkt der Okklusion. Ab diesem Zeitpunkt dann wird das geförderte Volumen erfasst, insbesondere die getätigten Motorschritte. Somit wird das geförderte Volumen bzw. werden die Motorschritte seit dem (geschätzten) Auftrittszeitpunkt der Okklusion erfasst, ohne dass der genaue Auftrittszeitpunkt der Okklusion ermittelt werden muss. Die Okklusionswahrscheinlichkeit wird in Echtzeit (also zeitaktuell) basierend auf dem erfassten Druckverlauf ausgegeben. Durch die Erfassung des Volumens, das zwischen dem (geschätzten) Auftrittszeitpunkt der Okklusion und dem Detektionszeitpunkt der Okklusion gefördert wurde, insbesondere die Erfassung der getätigten Motorschritte oder des Drehwinkels des Pumpenmotors, kann genau dieses erfasste Volumen zurückgepumpt werden bzw. die Motorschritte oder der Drehwinkel zurückgefahren werden. Als Folge wird der Bolus komplett wieder abgebaut oder zumindest stark minimiert. Eine gefährliche Verabreichung einer großen Menge Medikamente an den Patienten auf einmal wird dadurch gänzlich unterbunden oder ist zumindest deutlich unwahrscheinlicher.

Durch das offenbarungsgemäße Verfahren ist die Ermittlung des Bolusvolumens unabhängig von dem klinischen Aufbau, der Materialcharakteristik der Wegwerfartikel, insbesondere der Geometrie und des Materials, den Umweltbedingungen wie der Temperatur und des Ortes der Okklusion. Dadurch kann der (ungewollte) Bolus reduziert werden und die Sicherheit für den Patienten erhöht werden.

Die Aufgabe der vorliegenden Offenbarung wird ferner durch ein Prognoseverfahren zur Ermittlung der Okklusionswahrscheinlichkeit gelöst. Das Prognoseverfahren weist die folgenden Schritte auf. Die Sensoreinheit erfasst (historische) Druckverläufe. Zugehörige (Historische) Auftritte von Okklusionsereignissen werden ebenfalls erfasst. Die erfassten Druckverläufe und die erfassten Auftritte der Okklusionsereignisse werden zu einem Trainingsdatensatz kombiniert. Das System für maschinelles Lernen wird mit dem Trainingsdatensatz trainiert. Dabei sind insbesondere die erfassten (historischen) Druckverläufe die Eingangswerte und die Auftritte von (historischen) Okklusionsereignissen die Ausgangswerte des Systems für maschinelles Lernen. Während eines Pumpenbetriebs wird der (aktuelle) Druckverlauf erfasst und in Echtzeit in das trainierte System für maschinelles Lernen eingegeben. Das trainierte System für maschinelles Lernen (als Prognoseverfahren) gibt, basierend auf dem eingegebenen zeitaktuellen Druckverlauf, eine Schätzung/Vorhersage der Okklusionswahrscheinlichkeit in Echtzeit aus.

Das Prognoseverfahren kann im Wesentlichen in zwei unterschiedliche Phasen aufgeteilt werden. In einer Trainingsphase wird das System für maschinelles Lernen mit historischen Daten trainiert. Die historischen Daten wurden dabei in der Vergangenheit bei Testläufen oder schon abgeschlossenen Pumpvorgängen bzw. Infusionen erfasst. Wichtig ist dabei, dass einem erfassten Druckverlauf immer eine Information zugeordnet ist, ob es zu einer Okklusion gekommen ist oder nicht. Wenn die Information mit dem erfassten Druckverlauf und die Information über das Okklusionsauftreten zu einem zugeordneten Datensatz verknüpft sind, kann das System für maschinelles Lernen mit diesem Datensatz trainiert werden. Dadurch kann das System für maschinelles Lernen den Zusammenhang zwischen einem bestimmten Druckverlauf und dem Auftreten der Okklusion in Verbindung bringen und, beispielsweise über eine Korrelation eines neuen Druckverlaufs mit einem oder mehreren Druckverläufen und innerhalb eines Toleranzbereichs etwa, eine Wahrscheinlichkeit bestimmen, dass dieser Druckverlauf mit einer Okklusion einhergeht. Beispielsweise kann anhand einer Abweichung der Druckkurve eine Wahrscheinlichkeit angegeben werden. Damit lernt das System für maschinelles Lernen eine Okklusionswahrscheinlichkeit für den bestimmten Druckverlauf. Das trainierte System für maschinelles Lernen ist der Ausgangspunkt für eine zweite Phase, der Prognosephase. In der Prognosephase wird ein aktuell erfasster Druckverlauf in das trainierte System für maschinelles Lernen eingegeben. Das trainierte System für maschinelles Lernen gibt zu diesem Druckverlauf in Echtzeit die Okklusionswahrscheinlichkeit bzw. eine Aussage/Schätzung/Vorhersage, ob eine Okklusion auftreten wird oder nicht, aus. Durch Ausgabe der Okklusionswahrscheinlichkeit durch das System für maschinelles Lernen kann der Betriebsmodus der medizinischen Pumpe von dem Betriebsmodus mit hoher Okklusionswahrscheinlichkeit zu dem Betriebsmodus mit niedriger Okklusionswahrscheinlichkeit oder andersherum geändert werden. Der Wechsel des Betriebsmodus von niedriger zu hoher Okklusionswahrscheinlichkeit ist insbesondere eine Schätzung des Auftrittszeitpunktes der Okklusion.

Die Ausgabe des Systems für maschinelles Lernen kann insbesondere eine Prozentzahl sein, die die Okklusionswahrscheinlichkeit angibt. Beispielsweise kann ein Grenzwert festgelegt werden, wobei die Okklusionswahrscheinlichkeit eine hohe Okklusionswahrscheinlichkeit ist, wenn der Grenzwert überschritten wird. Dieser Grenzwert ist beispielsweise bei 50%. Das System für maschinelles Lernen kann auch einen binären Wert ausgeben, wobei eine "Eins" für eine hohe Okklusionswahrscheinlichkeit und eine "Null" für eine niedrige Okklusionswahrscheinlichkeit oder andersherum steht.

Die Aufgabe der vorliegenden Offenbarung wird ferner durch eine Sicherheitsvorrichtung/Sicherungsvorrichtung/Sicherheitseinheit/Sicherheitsmodul zur Reduzierung von dem (ungewollten) Bolus bzw. der ungewollten Ansammlung an Fluid in der Fluidleiteinheit, insbesondere einer Ausgangsleitung, einer medizinischen Pumpe, vorzugsweise einer Infusionspumpe, gelöst. Die (Sicherheits-)Vorrichtung weist eine Sensoreinheit zur Erfassung eines Druckverlaufs in der Fluidleiteinheit, insbesondere eine Speichereinheit zur Speicherung des erfassten Druckverlaufs und eine Detektionseinheit zur Detektion eines Auftretens einer Okklusion auf. Ferner weist die offenbarungsgemäße Vorrichtung eine Steuereinheit zur Ermittlung der Okklusionswahrscheinlichkeit und zur Erfassung eines von der Pumpe geförderten Volumens für einen Betriebsbereich mit hoher Okklusionswahrscheinlichkeit auf, insbesondere einer Anzahl an Motorschritten eines Pumpenmotors oder eines Drehwinkels des Pumpenmotors.

Die Steuereinheit gibt ferner das (Steuer-)Signal zum Zurückfahren der ermittelten Motorschritte oder der Anzahl Umdrehungen oder des Drehwinkels an den Pumpenmotor. Auf diese Weise wird, ganz analog zu dem Verfahren der vorliegenden Offenbarung, individuell das (geschätzte) geförderte Volumen ermittelt und entsprechend zurückbefördert, um den Bolus gänzlich abzubauen.

Vorteilhafte Weiterbildungen der vorliegenden Offenbarung sind Gegenstand der beigefügten Unteransprüche und werden insbesondere nachstehend erläutert.

Nach einem optionalen Aspekt der vorliegenden Offenbarung werden zur Ermittlung der Okklusionswahrscheinlichkeit die folgenden Schritte getätigt. Vorzugsweise wird ein (trainiertes) System für maschinelles Lernen mit dem erfassten Druckverlauf als Eingangswert und der Okklusionswahrscheinlichkeit als Ausgangswert erstellt. Der durch die Sensoreinheit erfasste Druckverlauf kann in Echtzeit in das (zuvor trainierte) System für maschinelles Lernen eingegeben werden. Das System für maschinelles Lernen gibt daraufhin die Okklusionswahrscheinlichkeit basierend auf dem erfassten und eingegebenen Druckverlauf aus. Das System für maschinelles Lernen kann dadurch einen Zusammenhang zwischen dem Druckverlauf und der Okklusionswahrscheinlichkeit herstellen. Basierend auf diesem Zusammenhang bzw. eines Wissens über eine Korrelation zwischen dem Druckverlauf und der Okklusionswahrscheinlichkeit kann das System für maschinelles Lernen die Okklusionswahrscheinlichkeit in Abhängigkeit des erfassten Druckverlaufs ausgeben. Die Ausgabe der Okklusionswahrscheinlichkeit erfolgt vorzugsweise in Echtzeit. Durch die Ausgabe der Okklusionswahrscheinlichkeit kann der Wechsel zwischen dem Betriebsmodus der Pumpe mit der niedrigen Okklusionswahrscheinlichkeit und dem Betriebsmodus der Pumpe mit der hohen Okklusionswahrscheinlichkeit ermittelt werden. Dadurch kann die Erfassung des geförderten Volumens, insbesondere der Motorschritt bzw. des Drehwinkels, gestartet werden. Mit dem Wechsel zwischen dem Betriebsmodus mit niedriger Okklusionswahrscheinlichkeit und dem Betriebsmodus mit hoher Okklusionswahrscheinlichkeit wird vorzugsweise eine Schätzung über das Auftreten bzw. den Auftrittszeitpunkt der Okklusion abgegeben.

Bei dem System für maschinelles Lernen handelt es sich vorzugsweise um ein künstliches neuronales Netz. Besonders bevorzugt können neuronale Netze verwendet werden, die für die Verarbeitung zeitabhängiger Datensätze entwickelt wurden, wie beispielsweise Recurrent neural networks oder LSTM-Netze (long short-term memory).

Vorzugsweise werden zum Erstellen des Systems für maschinelles Lernen die folgenden Schritte getätigt. Die Sensoreinheit erfasst den Druckverlauf. Dabei werden insbesondere (historische) Druckverläufe erfasst. Ein Auftreten von Okklusionsereignissen kann (durch die Steuereinheit) erfasst werden. Die erfassten Druckverläufe und die erfassten Auftritte der Okklusionsereignisse werden zu einem Trainingsdatensatz kombiniert. Das System für maschinelles Lernen kann mit dem Trainingsdatensatz trainiert werden. Dabei werden insbesondere die erfassten (historischen) Druckverläufe als Eingangswerte und die Auftritte von (historischen) Okklusionsereignissen als Ausgangswerte des Systems für maschinelles Lernen verwendet.

Aus (historisch erfassten) Druckverläufen der Pumpe und (historisch erfassten) Okklusionsevents/Okklusionsauftritten kann der Trainingsdatensatz erstellt werden. Vorzugsweise sind die Druckverläufe der Input/die Eingabe und das Auftreten der Okklusionsevents der Output/die Ausgabe des Systems für maschinelles Lernen. Das System für maschinelles Lernen lernt durch das Training den Zusammenhang zwischen den Druckverläufen und dem Auftreten von Okklusionen. Dadurch kann das System für maschinelles Lernen die Okklusionswahrscheinlichkeit in Abhängigkeit von dem Druckverlauf lernen. Wenn während des Betriebs der medizinischen Pumpe der Druckverlauf in der Fluidleiteinheit erfasst wird und in das System für maschinelles Lernen eingegeben wird, kann das trainierte System für maschinelles Lernen in Echtzeit die Okklusionswahrscheinlichkeit für den Druckverlauf ausgeben. Die Steuereinheit der Pumpe kann so zwischen einem Modus mit einer niedrigen und einem Modus mit einer hohen Okklusionswahrscheinlichkeit unterscheiden. Somit ist das trainierte System für maschinelles Lernen die Grundlage für die gezielte Erfassung der getätigten Motorschritte oder des Drehwinkels.

Nach einem weiteren optionalen Aspekt der vorliegenden Offenbarung werden zum Erfassen des von der Pumpe geförderten Volumens durch die Steuereinheit die folgenden Schritte getätigt. Vorzugsweise wird ein Zeitpunkt des Auftretens der Okklusion ermittelt. Die Steuereinheit kann eine Zeitspanne zwischen dem Auftrittszeitpunkt der Okklusion und dem Detektionszeitpunkt der Okklusion ermitteln. Das Volumen, das zwischen dem Auftrittszeitpunkt der Okklusion und dem Detektionszeitpunkt der Okklusion gefördert wurde, wird vorzugsweise aus der ermittelten Zeitspanne ermittelt.

Die Steuereinheit kann vorzugsweise durch eine retrospektive Betrachtung des erfassten Druckverlaufs nach der Detektion der Okklusion den Zeitpunkt des Auftretens des Bolus ermitteln. Aus der Zeitspanne zwischen dem Auftrittszeitpunkt der Okklusion und dem Detektionszeitpunkt der Okklusion kann die Steuereinheit mit Kenntnis einer Förderrate der Pumpe das Volumen berechnen, das zwischen dem Auftreten der Okklusion und der Detektion der Okklusion gefördert wurde. Dieses Volumen kann anschließend durch die Pumpe zurückgepumpt werden. Durch diese Ermittlung des gepumpten/geförderten Volumens kann die Pumpe den Bolus zielgerichtet reduzieren.

Insbesondere kann die Steuereinheit dafür angepasst sein, das geförderte Volumen zwischen dem Auftrittszeitpunkt und dem Detektionszeitpunkt durch die Anzahl der Motorschritte oder des Drehwinkels zu bestimmen, etwa indem das geförderte Volumen aufgezeichnet und in einer Speichereinheit hinterlegt ist, und dieses geförderte Volumen dann entsprechend zurückgepumpt werden.

Vorzugsweise werden zum Zurückpumpen des von der Pumpe geförderten Volumens durch die Pumpe zusätzlich die folgenden Schritte getätigt. Der Druck in der Fluidleiteinheit, insbesondere der Ausgangsleitung wird beispielsweise durch Zurückpumpen mit der Pumpe reduziert. Parallel/gleichzeitig wird der Druckverlauf durch die Sensoreinheit erfasst. Wenn der Druck in der Fluidleiteinheit ein Druckniveau erreicht, bei dem der Druck bei weiterem Zurückpumpen konstant ist, wird das Zurückpumpen gestoppt bzw. der Pumpenmotor wird gestoppt, um ein Zurückpumpen sicher zu stoppen und ein Überpumpen zu verhindern. Das Vorhandensein des Bolus kann ein erhöhtes Druckniveau in der Fluidleiteinheit bedeuten. Wenn das angesammelte Fluid abgepumpt wird, reduziert sich der Druck in der Fluidleiteinheit. Wenn der Bolus abgepumpt ist, folgt aus weiterem Pumpen keine Druckreduzierung mehr. Wenn also gepumpt wird und der Druck in der Fluidleiteinheit trotz Pumpen nicht absinkt, kann davon ausgegangen werden, dass der Bolus reduziert bzw. abgepumpt wurde. Beispielsweise kann dies auf eine elastische Ausdehnung des Schlauchs zurückzuführen sein, der sich jedoch zu einem Grad weit irreversibel ausgedehnt hat und damit (ohne Überdruck) ein größeres Volumen als zuvor aufweist. Daraufhin wird der Pumpenmotor gestoppt. Durch das Abpumpen mit paralleler Druckerfassung kann der Bolus gezielt abgepumpt werden. Dadurch wird vorzugsweise eine (redundante) Sicherung realisiert, die verhindert, dass zu viel Fluid abgepumpt wird.

Vorzugsweise ermittelt die Steuereinheit den Zeitpunkt des Auftretens der Okklusion, indem die Steuereinheit den erfassten Druckverlauf zweimal ableitet und die Nullstellen der zweiten Ableitung des erfassten Druckverlaufs ermittelt und von diesen Nullstellen die Nullstelle mit einem Minimalwert auswählt. Der erfasste Druck ist zeitabhängig und ergibt eine Kurve in einem Druck-Zeit-Diagramm oder in einem DruckVolumen-Diagramm, wobei das Volumen das Volumen ist, das von der Pumpe gefördert wurde. Das Auftreten der Okklusion ändert den Druckverlauf. So können durch eine Betrachtung der Extrema und/oder der Wendepunkte des Druckverlaufs die Stellen ermittelt werden, an denen sich der Druck ändert.

Vorzugsweise ermittelt die Steuereinheit den Zeitpunkt des Auftretens der Okklusion, indem die Steuereinheit den erfassten Druckverlauf einfach ableitet und eine Änderung der Steigung des erfassten Druckverlaufs ermittelt. Insbesondere ermittelt die Steuereinheit einen Minimalwert, an dem die Steigung ansteigt. Durch die Okklusion ändert sich der Druckverlauf. Der Druck steigt als Folge der Okklusion. Die Steuereinheit ermittelt den Zeitpunkt, ab dem der Druck ansteigt. Dieser Zeitpunkt kann den Zeitpunkt des Auftretens der Okklusion darstellen.

Vorzugsweise ermittelt die Steuereinheit den Zeitpunkt des Auftretens der Okklusion, indem die Steuereinheit Wendepunkte des erfassten Druckverlaufs ermittelt, wobei ein kleinster Wendepunkt (ein Wendepunkt zu einem kleinsten Druck) den ermittelten Auftrittszeitpunkt der Okklusion darstellt.

Die Aufgabe der vorliegenden Offenbarung wird ferner durch eine medizinische Pumpe mit einer Steuereinheit gelöst, die ausgebildet und vorbereitet ist, ein Verfahren gemäß den vorstehenden Aspekten auszuführen.

Insbesondere kann die medizinische Pumpe, insbesondere Infusionspumpe, eine Sicherungsvorrichtung zur Reduzierung von einem Bolus gemäß der vorliegenden Offenbarung aufweisen.

Jegliche Offenbarung im Zusammenhang mit dem Verfahren gemäß der vorliegenden Offenbarung gilt ebenso für die Sicherheitsvorrichtung sowie die medizinische Pumpe sowie auch jegliche Offenbarung im Zusammenhang mit der Sicherheitsvorrichtung und der medizinischen Pumpe der vorliegenden Offenbarung auch für das Verfahren der vorliegenden Offenbarung gilt. Insbesondere kann die Steuereinheit der Sicherheitsvorrichtung bzw. der medizinischen Pumpe entsprechend angepasst sein, die Verfahrensschritte des Verfahrens auszuführen.

### Kurzbeschreibung der Figuren

Die Offenbarung wird nachfolgend anhand bevorzugter Ausführungsformen mithilfe von Figuren näher erläutert. Es zeigen:
- Fig. 1: eine perspektivische Ansicht einer medizinischen Pumpe gemäß einer bevorzugten Ausführungsform;
- Fig. 2: eine schematische Darstellung einer Sicherungsvorrichtung gemäß einer bevorzugten Ausführungsform;
- Fig. 3: ein Ablaufdiagramm eines offenbarungsgemäßen Verfahrens gemäß einer bevorzugten Ausführungsform zur Reduzierung eines Bolus;
- Fig. 4: ein Ablaufdiagramm eines offenbarungsgemäßen Prognoseverfahrens einer bevorzugten Ausführungsform;
- Fig. 5: eine schematische Darstellung eines Systems für maschinelles Lernen; und
- Fig. 6: ein Diagramm zur Veranschaulichung der Funktionsweise der vorliegenden Offenbarung mit einem Zusammenhang zwischen einem geförderten Volumen der medizinischen Pumpe und einem Druck.

Die Figuren sind schematischer Natur und sollen nur dem Verständnis der Offenbarung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

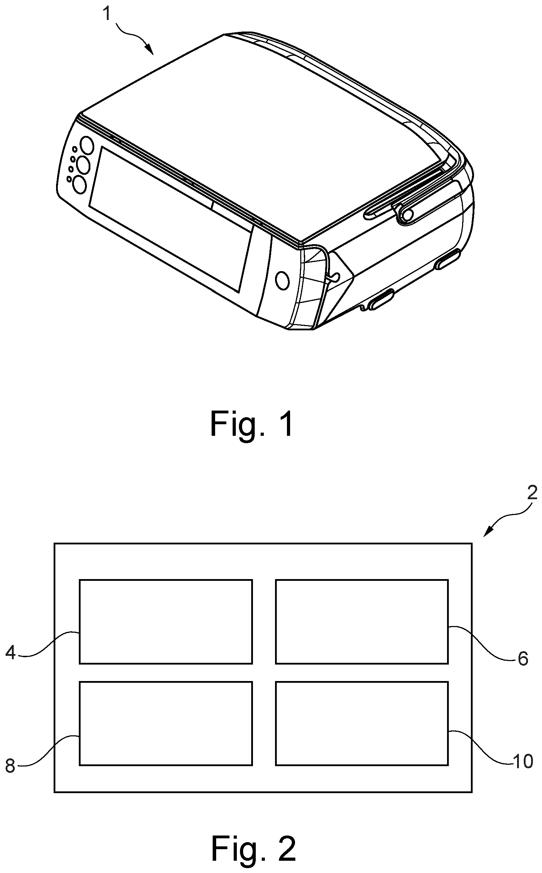

Fig. 1 zeigt in einer perspektivischen Ansicht eine Infusionspumpe 1. Die Infusionspumpe 1 weist eine Fluidleiteinheit/eine Ausgangsleitung (nicht dargestellt) auf, die zu einem Patienten (nicht dargestellt) führt. Diese Ausgangsleitung ist in eine Front der Infusionspumpe 1 eingelegt und wird durch eine Pumpe entsprechend aktuiert. Die Ausgangsleitung ist als ein Wegwerfartikel aus einem elastischen Material ausgeführt und ist beispielsweise ein Schlauchsystem. Durch die Ausgangsleitung wird ein medizinisches Fluid, insbesondere ein Infusionsfluid, gefördert/gepumpt, in dem eine Medikation für den Patienten enthalten ist. Sollte es zu einer Verstopfung/Okklusion in der Ausgangsleitung kommen, kann sich in der elastischen Ausgangsleitung eine (ungewollte) Ansammlung des Fluides, der sogenannte Bolus, akkumulieren. Wenn die Okklusion gelöst wird, könnte dadurch der Bolus auf einmal an den Patienten abgegeben werden. Das ist besonders bei kritischen Medikamenten zu vermeiden. Deshalb weist die Infusionspumpe 1 der vorliegenden Ausführungsform eine Sicherheitsvorrichtung 2 zur Reduzierung des Bolus in der Ausgangsleitung auf bzw. kann auf der Infusionspumpe 1 ein offenbarungsgemäßes Verfahren zur Reduzierung des Bolus ausgeführt werden, wie nachstehend zu Fig. 2 bis 4 erläutert wird.

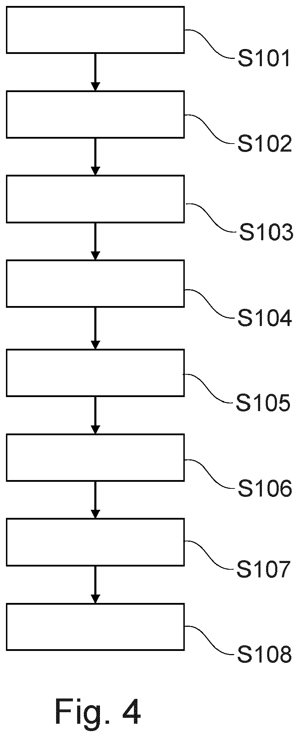

Fig. 2 zeigt eine schematische Darstellung einer Konfiguration einer solchen Sicherheitsvorrichtung 2, welche in der Infusionspumpe 1 eingesetzt ist. Die Sicherheitsvorrichtung 2 weist eine Sensoreinheit 4, eine Speichereinheit 6, eine Steuereinheit 8 und eine Detektionseinheit 10 auf. Die Sensoreinheit 4 erfasst hierbei einen Druckverlauf in der Ausgangsleitung. Die Speichereinheit 6 speichert den durch die Sensoreinheit 4 erfassten Druckverlauf. Die Steuereinheit 8 ermittelt eine Okklusionswahrscheinlichkeit, insbesondere gemäß einem Prognoseverfahren zur Ermittlung der Okklusionswahrscheinlichkeit, welches nachstehend im Zusammenhang mit Fig. 4 detailliert erläutert wird, und erfasst ein von der Pumpe 1 gefördertes Volumen für einen Betriebsbereich mit (bzw. zeitlich ab) einer hohen Okklusionswahrscheinlichkeit. Damit wird auch ein geschätzter Auftrittszeitpunkt der Okklusion bestimmt. Insbesondere erfasst die Steuereinheit 8 eine Anzahl an Motorschritten eines Pumpenmotors (nicht dargestellt) oder eines Drehwinkels des Pumpenmotors. Die Detektionseinheit 10 detektiert ein Auftreten einer Okklusion zu einem Detektionszeitpunkt in der Ausgangsleitung, wenn der erfasste Druck einen vordefinierten Druckgrenzwert überschreitet.

Basierend auf der Ermittlung der ausgeführten Motorschritte sendet die Steuereinheit ein (Steuer-)Signal an den Pumpenmotor, das die ausgeführten Motorschritte zurückgefahren werden. Hierdurch wird der durch die Verstopfung ungewollt erzeugte Bolus individuell und sicher wieder entfernt. Es wird damit eine einfache und zuverlässige Sicherheitsvorrichtung 2 sowie Infusionspumpe 1 bereitgestellt, welche sicher und genau einen Zustand vor Auftreten der Okklusion herstellen kann.

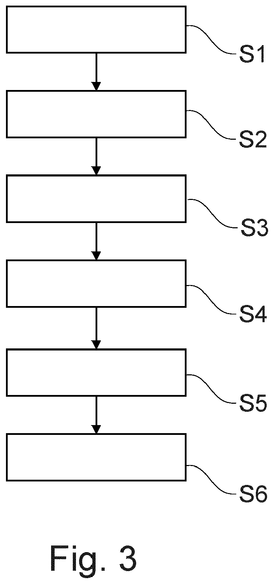

Fig. 3 zeigt ein Ablaufdiagramm eines Verfahrens gemäß einer bevorzugten Ausführungsform der vorliegenden Offenbarung zur Reduzierung eines Bolus in der Ausgangsleitung der Infusionspumpe 1. Die Infusionspumpe kann insbesondere über entsprechend angepasste Steuereinheit dafür adaptiert sein, das Verfahren auszuführen.

In Schritt S1 wird zunächst ein Druckverlauf in der Ausgangsleitung durch die Sensoreinheit 4 erfasst. Es wird ein zeitlicher Verlauf erstellt.

In einem Schritt S2 wird der erfasste Druckverlauf in der Speichereinheit 6 gespeichert, um auf diesen auch wieder zugreifen und den zeitlichen Verlauf analysieren zu können.

In Schritt S3 ermittelt die entsprechend angepasste Steuereinheit 8 eine Okklusionswahrscheinlichkeit basierend auf dem erfassten und gespeicherten Druckverlauf.

In Schritt S4 erfasst die Steuereinheit 10 ein von der Pumpe gefördertes Volumen für einen Betriebsbereich mit hoher Okklusionswahrscheinlichkeit (bzw. ab einem geschätzten Auftrittszeitpunkt), insbesondere eine Anzahl an Motorschritten des Pumpenmotors oder den Drehwinkel des Pumpenmotors.

In Schritt S5 detektiert die Detektionseinheit 8 das Auftreten der Okklusion zu einem Detektionszeitpunkt.

In Schritt S6 wird das für den ermittelten Betriebsbereich mit hoher Okklusionswahrscheinlichkeit erfasste von der Pumpe geförderte Volumen durch die Pumpe zurückgepumpt. Insbesondere werden die erfassten Motorschritte oder der erfasste Drehwinkel zurückgefahren. Damit wird der Bolus reduziert und gänzlich abgebaut.

Vorzugsweise kann redundant hierzu das Verfahren den Schritt aufweisen, Zeitaktuelles Erfassen des Druckes durch die Sensoreinheit und Stoppen eines Zurückpumpen, falls ein vordefiniertes Druckniveau, insbesondere ein Druckniveau vor Bestimmung eines Betriebsbereichs einer hohen Okklusionswahrscheinlichkeit, erreicht wurde, um ein übermäßiges Zurückpumpen zu verhindern.

Fig. 4 zeigt ein Ablaufdiagramm eines Prognoseverfahrens gemäß einer bevorzugten Ausführungsform zur Ermittlung der Okklusionswahrscheinlichkeit. Das Prognoseverfahren kann in eine Trainingsphase und eine Prognosephase unterteilt werden.

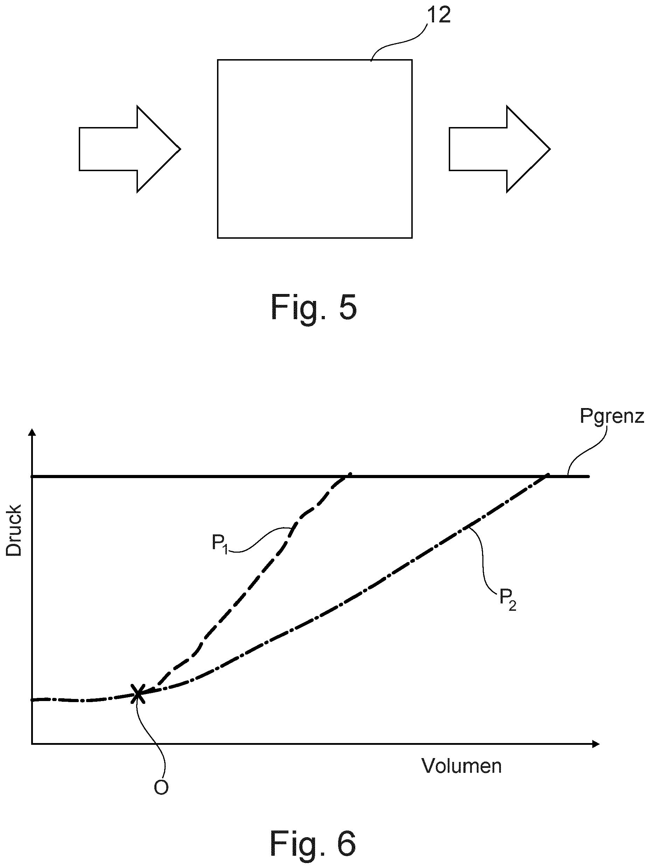

In der Trainingsphase wird ein System für maschinelles Lernen 12 (in Fig. 5 gezeigt) trainiert.

In Schritt S101 werden (historische) Druckverläufe durch die Sensoreinheit 4 erfasst.

In Schritt S102 werden Auftritte von Okklusionsereignissen bei den erfassten Druckverläufen erfasst.

In Schritt S103 werden die erfassten Druckverläufe und die erfassten Auftritte der Okklusionsereignisse zu einem (verknüpften) Trainingsdatensatz kombiniert.

In Schritt S104 wird das System für maschinelles Lernen mit einem erfassten Druckverlauf als Eingangswert und einem Auftreten der Okklusionsereignisse als Ausgangswert erstellt.

In Schritt S105 wird das System für maschinelles Lernen 12 mit dem Trainingsdatensatz trainiert. Das trainierte System für maschinelles Lernen 12 ist das Endresultat der Trainingsphase und der Ausgangspunkt der Prognosephase.

In der Prognosephase wird das System für maschinelles Lernen 12 nicht mehr mit historischen Daten trainiert, sondern das System für maschinelles Lernen 12 soll bei aktuellen Daten eine Prognose/Schätzung abgeben.

In Schritt S106 erfasst die Sensoreinheit 4 einen (aktuellen) Druckverlauf in Echtzeit.

Der erfasste Druckverlauf wird in Schritt S107 in das trainierte System für maschinelles Lernen 12 eingegeben.

Basierend auf dem eingegebenen Druckverlauf gibt das trainierte System für maschinelles Lernen 12 die Okklusionswahrscheinlichkeit in Schritt S108 aus.

Auf diese Weise kann eine Prognose in Form einer Prozentualen Wahrscheinlichkeit/Schätzung einer Okklusion bereitgestellt werden.

Fig. 5 zeigt eine schematische Darstellung des Systems für maschinelles Lernen 12. Das System für maschinelles Lernen 12 ist vorzugsweise ein (künstliches) neuronales Netz und weist eine Eingangsschicht und eine Ausgangsschicht auf. In die Eingangsschicht werden Eingangswerte eingegeben. Die Eingangswerte sind im vorliegenden Fall ein Druckverlauf. Aus der Ausgangsschicht wird ein Ausgangswert ausgegeben. Der Ausgangswert ist die Okklusionswahrscheinlichkeit.

Das System für maschinelles Lernen 12 bildet also einen Zusammenhang zwischen einem Druckverlauf als Eingangswert und einer dazu korrelierenden Okklusionswahrscheinlichkeit als Ausgangswert ab.

Fig. 6 zeigt zur Veranschaulichung der Funktion der vorliegenden Offenbarung ein Diagramm, das einen Zusammenhang zwischen dem geförderten Volumen der Pumpe 1 und einem Druck in der Ausgangsleitung zeigt. In einem ersten Bereich ist der Druck im Wesentlichen konstant. Das heißt, die Pumpe fördert kontinuierlich ein Volumen und der Druck in der Ausgangsleitung bleibt entsprechend konstant bzw. auf gleichem Druckniveau, da das geförderte Fluid ungehindert abfließen kann. Es bildet sich sozusagen ein Fluidstrom. Wenn eine Okklusion in der Ausgangsleitung auftritt (Auftrittszeitpunkt der Okklusion), steigt der Druck entsprechend an, wenn weiteres Fluid von der Pumpe in die Ausgangsleitung gefördert wird. Der Druck steigt solange an, bis ein Druckgrenzwert P_{grenz} überschritten wird. Wird der Druckgrenzwert P_{grenz} (Detektionszeitpunkt der Detektion der Okklusion) überschritten, dann wird ein Alarm ausgegeben und die Pumpe 1 gestoppt. Das Bolusvolumen ist dabei abhängig von der Steigung des Drucks nach dem Auftreten des Bolus.

Steigt der Druck nach dem Auftreten der Okklusion schnell an, wurde zwischen dem Auftreten der Okklusion und dem Überschreiten des Druckgrenzwertes als Alarmbedingung meist nur wenig Volumen gefördert (beispielsweise ist die Ausgangsleitung kurz und stellt kein träges, sondern ein schnell reagierendes System dar). Der Bolus hat dementsprechend ein kleines Volumen (Kurve P1). Steigt der Druck nach dem Auftreten der Okklusion hingegen langsamer an, wird zwischen dem Auftreten der Okklusion (bzw. geschätzten Auftrittszeitpunkt) und dem Überschreiten des Druckgrenzwertes (Detektionszeitpunkt) mehr Volumen gefördert, als bei einem schnellen Druckanstieg. Das Bolusvolumen ist somit größer (Kurve P2).

Aus der Kurve des Druckverlaufs kann durch retrospektive Analyse der Auftrittszeitpunkt der Okklusion ermittelt werden. Durch die retrospektive Betrachtung kann somit das Volumen ermittelt werden, das zwischen dem Auftrittszeitpunkt der Okklusion und dem Detektionszeitpunkt der Okklusion gefördert wurde.

Insbesondere kann der Auftrittszeitpunkt der Okklusion durch die folgenden Möglichkeiten ermittelt werden:
- Bilden der zweiten Ableitung des Druckverlaufs und Nullpunktanalyse der zweiten Ableitung;
- Analyse der Steigung des Druckverlaufs;
- Suche nach einem kleinsten Wendepunkt durch einen Suchalgorithmus.

Bei dem Auftreten einer Okklusion ändert sich die Steigung des Druckverlaufes. Also kann durch eine Analyse der Steigung oder einer Suche nach einem Punkt einer Steigerungsänderung der Auftrittszeitpunkt der Okklusion ermittelt werden. Dieses Wissen kann vorliegend dafür verwendet werden, um den Auftrittszeitpunkt der Okklusion zu bestimmen.

Bei der Steuereinheit 10 kann es sich um eine Computereinheit, einen Prozessor, ein Motorsteuergerät, einen Mikrocontroller oder Ähnliches handeln. Voraussetzung ist lediglich, dass die Steuereinheit 10 dazu vorbereitet und ausgebildet ist, das bzw. die offenbarungsgemäßen Verfahren auszuführen.

### Bezugszeichenliste

- 1: Infusionspumpe
- 2: Sicherheitsvorrichtung
- 4: Sensoreinheit
- 6: Speichereinheit
- 8: Steuereinheit
- 10: Detektionseinheit
- 12: System für maschinelles Lernen

- P1: erster Druck
- P2: zweiter Druck
- O: Okklusionsereignis
- Pgrenz: Druckgrenzwert

## Patentansprüche

1. Verfahren zur Reduzierung eines Bolus in einer Fluidleiteinheit, insbesondere in einer Ausgangsleitung, einer medizinischen Pumpe (1), vorzugsweise einer Infusionspumpe, mit den folgenden Schritten:
- Erfassen eines Druckverlaufs in der Fluidleiteinheit durch eine Sensoreinheit (4);
- Vorzugsweise Speichern des erfassten Druckverlaufs in einer Speichereinheit (6);
- Ermitteln einer Okklusionswahrscheinlichkeit durch eine Steuereinheit (8) basierend auf dem erfassten Druckverlauf;
- Erfassen eines von der Pumpe (1) geförderten Volumens für einen Betriebsbereich mit hoher Okklusionswahrscheinlichkeit, insbesondere einer Anzahl an Motorschritten eines Pumpenmotors oder eines Drehwinkels des Pumpenmotors, durch die Steuereinheit (8);
- Detektieren eines tatsächlichen Auftretens einer Okklusion zu einem Detektionszeitpunkt durch eine Detektionseinheit (10) bei Überschreiten einer Alarmbedingung, insbesondere eines Druckgrenzwertes als Alarmbedingung; und
- Zurückpumpen des für den ermittelten Betriebsbereich mit hoher Okklusionswahrscheinlichkeit erfassten von der Pumpe (1) geförderten Volumens durch die Pumpe (1), insbesondere durch Zurückfahren der erfassten Motorschritte oder des erfassten Drehwinkels.

2. Verfahren nach Anspruch 1, **dadurch gekennzeichnet, dass** zur Ermittlung der Okklusionswahrscheinlichkeit die folgenden Schritte durchgeführt werden:
- Erstellen eines trainierten Systems für maschinelles Lernen (12) mit dem erfassten Druckverlauf als Eingangswert und der Okklusionswahrscheinlichkeit als Ausgangswert;
- Eingabe des durch die Sensoreinheit (4) erfassten Druckverlaufs in Echtzeit in das zuvor trainierte System für maschinelles Lernen (12); und
- Ausgabe der Okklusionswahrscheinlichkeit in Echtzeit durch das trainierte System für maschinelles Lernen (12) basierend auf dem erfassten und eingegebenen Druckverlauf.

3. Verfahren nach Anspruch 2, **dadurch gekennzeichnet, dass** zum Erstellen des Systems für maschinelles Lernen (12) die folgenden Schritte durchgeführt werden:
- Erfassen des Druckverlaufs durch die Sensoreinheit (4);
- Erfassen des Auftretens von Okklusionsereignissen;
- Kombinieren des erfassten Druckverlaufs und des erfassten Auftretens der Okklusionsereignisse zu einem Trainingsdatensatz; und
- Trainieren des Systems für maschinelles Lernen (12), insbesondere eines neuronalen Netzes, mit dem erfassten Druckverlauf als Eingangswert und dem Auftreten der Okklusionsereignisse als Ausgangswert.

4. Verfahren nach einem der Ansprüche 1 bis 3, **dadurch gekennzeichnet, dass** das Erfassen des von der Pumpe (1) geförderten Volumens durch die Steuereinheit (8) die folgenden Schritte aufweist:
- Ermitteln eines Auftrittszeitpunkts der Okklusion, insbesondere eines Zeitpunkts eines Beginns eines Betriebsbereichs mit hoher Okklusionswahrscheinlichkeit;
- Ermitteln einer Zeitspanne und/oder einer Anzahl an Umdrehungen der Pumpe (1) zwischen dem Auftrittszeitpunkt der Okklusion und dem Detektionszeitpunkt der Okklusion durch die Steuereinheit (8); und
- Ermitteln des Volumens, das zwischen dem Auftrittszeitpunkt der Okklusion und dem Detektionszeitpunkt der Okklusion gefördert wurde, aus der ermittelten Zeitspanne und/oder der Anzahl der Umdrehungen durch die Steuereinheit (8).

5. Verfahren nach einem der Ansprüche 1 bis 4, **dadurch gekennzeichnet, dass** das Zurückpumpen des von der Pumpe (1) geförderten Volumens durch die Pumpe (1) zusätzlich die folgenden Schritte aufweist:
- Reduzieren des Drucks durch Zurückpumpen des Fluides durch die Pumpe (1) und gleichzeitiges Erfassen des Druckverlaufs durch die Sensoreinheit (4); und
- Stoppen des Zurückpumpens, wenn der Druck ein Druckniveau erreicht, bei dem der Druck bei weiterem Pumpen konstant ist, um ein Zurückpumpen sicher zu stoppen und ein Überpumpen zu verhindern.

6. Prognoseverfahren zur Ermittlung einer Okklusionswahrscheinlichkeit mit den folgenden Schritten:
- Erfassen von Druckverläufen durch eine Sensoreinheit (4);
- Erfassen von Auftritten von Okklusionsereignissen bei den zugehörigen erfassten Druckverläufen;
- Kombinieren der erfassten Druckverläufe und der erfassten Auftritte der Okklusionsereignisse zu einem Trainingsdatensatz;
- Erstellen eines Systems für maschinelles Lernen (12) mit einem erfassten Druckverlauf als Eingangswert und einem Auftreten der Okklusionsereignisse als Ausgangswert;
- Trainieren des Systems für maschinelles Lernen (12) mit dem Trainingsdatensatz;
- Erfassen eines Druckverlaufs in Echtzeit durch die Sensoreinheit (4);
- Eingabe des in Echtzeit erfassten Druckverlaufs in das trainierte System für maschinelles Lernen (12); und
- Ausgabe einer Schätzung einer Okklusionswahrscheinlichkeit in Echtzeit, basierend auf dem eingegebenen Druckverlauf durch das trainierte System für maschinelles Lernen (12).

7. Sicherheitsvorrichtung (2) zur Reduzierung eines Bolus in einer Fluidleiteinheit einer medizinischen Pumpe (1), vorzugsweise einer Infusionspumpe, aufweisend:
- eine Sensoreinheit (4), die dafür angepasst ist, einen Druckverlauf in der Fluidleiteinheit zu erfassen;
- vorzugsweise eine Speichereinheit (6), die dafür angepasst ist, den erfassten Druckverlauf zu speichern;
- eine Detektionseinheit (10), die dafür angepasst ist ein Auftreten einer Okklusion zu detektieren; und
- eine Steuereinheit (8), die dafür angepasst ist ein von der Pumpe (1) gefördertes Volumens für einen Betriebsbereich mit hoher Okklusionswahrscheinlichkeit zu erfassen, insbesondere einer Anzahl an Motorschritten eines Pumpenmotors oder eines Drehwinkels des Pumpenmotors.

8. Sicherheitsvorrichtung (2) nach Anspruch 7, **dadurch gekennzeichnet, dass** die Steuereinheit (8) dafür angepasst ist, den Auftrittszeitpunkt der Okklusion derart zu ermitteln, indem die Steuereinheit (8) den erfassten Druckverlauf zweimal ableitet und die Nullstellen der zweiten Ableitung des erfassten Druckverlaufs ermittelt und von diesen Nullstellen die Nullstelle mit einem Minimalwert auswählt.

9. Sicherheitsvorrichtung (2) nach Anspruch 7 oder 8, **dadurch gekennzeichnet, dass** die Steuereinheit (8) dafür angepasst ist, den Auftrittszeitpunkt der Okklusion zu ermitteln, indem die Steuereinheit (8) den erfassten Druckverlauf einfach ableitet und eine Änderung der Steigung des erfassten Druckverlaufs ermittelt zu einem Minimalwert, an dem die Steigung ansteigt.

10. Sicherheitsvorrichtung (2) nach Anspruch 7, **dadurch gekennzeichnet, dass** die Steuereinheit (8) dafür angepasst ist den Auftrittszeitpunkt der Okklusion zu ermitteln, indem die Steuereinheit (8) Wendepunkte des erfassten Druckverlaufs ermittelt, wobei ein kleinster Wendepunkt den Auftrittszeitpunkt der Okklusion darstellt.

11. Sicherheitsvorrichtung (2) nach Anspruch 7, **dadurch gekennzeichnet, dass** die Steuereinheit (8) dafür angepasst ist, die Okklusionswahrscheinlichkeit als hoch einzustufen, wenn die Okklusionswahrscheinlichkeit einen vordefinierten Grenzwert überschreitet, insbesondere ist die Sicherheitsvorrichtung (2) dafür angepasst, dass der vordefinierte Grenzwert vor dem Beginn einer Behandlung variabel einstellbar ist.

12. Medizinische Pumpe (1), insbesondere Infusionspumpe, mit einer Steuereinheit (8), die dafür vorgesehen und angepasst ist, ein Verfahren der Ansprüche 1 bis 5 auszuführen.

13. Medizinische Pumpe (1), insbesondere Infusionspumpe mit einer Sicherheitsvorrichtung (2) nach einem der Ansprüche 7 bis 10.
